# EUROPÄISCHE PATENTSCHRIFT

(11) **EP 1 363 586 B1**
(45) Veröffentlichungstag und Bekanntmachung des Hinweises auf die Patenterteilung: **25.02.2009**
(21) Anmeldenummer: 02700249.2
(22) Anmeldetag: 19.02.2002
(51) Int. Cl.: A61K 8/89, A61Q 5/04

(54) **VERFAHREN ZUR DAUERHAFTEN VERFORMUNG KERATINISCHER FASERN UND MITTEL**
METHOD FOR THE LONG-LASTING DEFORMATION OF KERATIN FIBRES AND CORRESPONDING PRODUCT
PROCEDE DE MISE EN FORME DURABLE DE FIBRES KERATINIQUE ET PRODUIT CORRESPONDANT

(30) Priorität: 28.02.2001 DE 10109730
(43) Veröffentlichungstag der Anmeldung: 26.11.2003
(73) Patentinhaber: Henkel AG & Co. KGaA, 40589 Düsseldorf (DE)
(72) Erfinder: KNAPPE, Thorsten, 22525 Hamburg (DE); MÜLLER, Burkhard, 21075 Hamburg (DE)
(86) Internationale Anmeldenummer: PCT/EP2002/001717
(87) Internationale Veröffentlichungsnummer: WO 2002/067881

(56) Entgegenhaltungen:
- WO-A-99/58099
- DE-A- 3 009 763
- DE-A- 19 534 722
- DE-C- 19 534 723
- US-A- 5 093 113

## Beschreibung

Die Erfindung betrifft ein Verfahren zur dauerhaften Verformung von Keratinfasern, insbesondere von menschlichen Haaren, durch reduktive Spaltung und erneute oxidative Knüpfung von Disulfidbindungen des Keratins sowie für dieses Verfahren geeignete Mittel.

Die dauerhafte Verformung von Keratinfasern wird üblicherweise so durchgeführt, daß man die Faser mechanisch verformt und die Verformung durch geeignete Hilfsmittel festlegt. Vor und/oder nach dieser Verformung behandelt man die Faser mit der wäßrigen Zubereitung einer keratinreduzierenden Substanz und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung des Keratinreduktionsmittels ist üblicherweise alkalisch eingestellt, damit zum einen genügender Anteil der Thiolfunktionen deprotoniert vorliegt und zum anderen die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so daß es zu einer Lockerung der Peptidvemetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Eine negative Begleiterscheinung der so durchgeführten Dauerwellung des Haares ist aber häufig ein Verspröden und Stumpfwerden der Haare. Weiterhin werden in vielen Fällen auch andere Eigenschaften wie Naß- und Trockenkämmbarkeit, Griff, Geschmeidigkeit, Weichheit, Glanz und Reißfestigkeit in ungewünschter Weise beeinflußt.

Es hat daher in der Vergangenheit nicht an Versuchen gefehlt, hier für Abhilfe zu sorgen.

Eine entsprechende Modifizierung der reduzierenden Lösung führt zu in der Regel nicht befriedigenden Welleistungen. Die Zugabe bekannter Zusätze wie Strukturanten, Polymere, Filmbildner und vernetzende Harze oder die neutrale bis schwach saure Einstellung der Zubereitung kann die Schädigung des Haares zwar verringern, jedoch bleibt das Haar in seiner Struktur mehr oder weniger geschwächt. Die pflegende Behandlung der Haare durch weitere Nachbehandlungen kann zwar die Haareigenschaften wieder verbessern, erfordert jedoch zusätzlichen Zeitaufwand und in der Regel die Verwendung mindestens eines weiteren Mittels.

Es bestand daher weiterhin die Aufgabe, ein Verfahren der dauerhaften Verformung von Keratinfasern zu finden, bei welchem die genannten unerwünschten Nebenwirkungen weiter reduziert oder ganz ausgeschlossen werden.

Es wurde nun überraschenderweise gefunden, daß eine wesentliche Verbesserung der Eigenschaften verformter Keratinfasern, wie verbesserte Kämmbarkeit und Avivage, dadurch erreicht wird, daß mindestens ein während des Verformungsverfahrens eingesetztes Mittel mehrphasig formuliert ist und spezielle Verbindungen enthält.

Gegenstand der Erfindung ist daher ein Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült und gegebenenfalls nachbehandelt, dadurch gekennzeichnet, daß mindestens eine der beiden wäßrigen Zubereitungen oder die erste Spülung in Form eines Zwei- oder Mehrphasensystems vorliegt, das mindestens eine hydrophobe Komponente gemäß Anspruch 1 enthält und das zum Aufbringen auf die Faser durch mechanische Bewegung in ein homogenes System überführt wird.

Im weiteren werden folgende Bezeichnungen verwendet:
- "Wellmittel" für die wäßrige Zubereitung der keratinreduzierenden Substanz,
- "Zwischenspülung" für die erste Spülung und
- "Fixiermittel" für die wäßrige Zubereitung des Oxidationsmittels.

Im erfindungsgemäßen Verfahren ist das Wellmittel, die Zwischenspülung und/oder das Fixiermittel in Form eines Zwei- oder Mehrphasensystems formuliert. Erfindungsgemäß eingesetzte Zwei- und Mehrphasensysteme sind Systeme, bei denen mindestens zwei separate, kontinuierliche Phasen vorliegen. Beispiele für solche Systeme sind Zubereitungen, die folgende Phasen aufweisen:
- eine wäßrige Phase und eine nichtwäßrige Phase, die separat voneinander vorliegen
- eine wäßrige Phase und zwei nichtwäßrige, miteinander nicht mischbare Phasen, die jeweils separat vorliegen
- eine Öl-in-Wasser-Emulsion und eine davon separiert vorliegende nichtwäßrige Phase
- eine Wasser-in-Öl-Emulsion und eine davon separiert vorliegende wäßrige Phase.

Keine Zwei-Phasensysteme im Sinne der vorliegenden Erfindung sind Systeme, bei denen nur eine kontinuierliche Phase vorliegt, wie z. B. reine Öl-in-Wasser- oder Wasser-in-Öl-Emulsionen.

Die im erfindungsgemäßen Verfahren eingesetzten, Zwei- oder Mehrphasensysteme aufweisenden Mittel entfalten ihre volle Wirkung aber nur dann, wenn sie in homogener Form auf die Keratinfaser aufgebracht werden. Dazu werden die Mittel durch mechanische Einwirkung, z. B. einfaches Schütteln des sie enthaltenden Behälters mit der Hand, in homogene Systeme überführt. Um ein homogenes Auftragen auf die Keratinfaser sicherzustellen, muß dieser homogene Zustand für eine ausreichende Zeit erhalten bleiben, bevor sich die einzelnen Phasen wieder ausbilden. Für die erfindungsgemäße Lehre hat es sich als ausreichend erwiesen, wenn dieser homogene Zustand mindestens 5 Sekunden, insbesondere mindestens 20 Sekunden, stabil ist, bevor für den Betrachter wieder eine Grenzschicht und somit die Ausbildung der einzelnen Phasen erkennbar wird.

Die erfindungsgemäß eingesetzten Zwei- und Mehrphasensysteme enthalten neben Wasser zwingend mindestens eine hydrophobe Komponente. Bevorzugt als hydrophobe Komponente sind ausgewählte Silikonöle. Dabei ist es erfindungsgemäß selbstverständlich möglich, die hydrophobe Komponente, insbesondere die ausgewählten Silikonöle, in der einen Phase und Alkohole und/oder Kohlenhydrate in der anderen Phase zu verwenden. Ganz besonders bevorzugt ist jedoch die Verwendung von zwei oder mehr hydrophoben Komponenten, wobei ganz besonders vorteilhaft mindestens eine der hydrophoben Komponenten ein erfindungsgemäß ausgewähltes Silikonöl und die andere hydrophobe Komponente mindestens ein Ölkörper oder Wachs ist.

In der Patentschrift US 5093113 werden hierzu saure Dauerwellmittel als Zweiphasengemische beschrieben, welche als hydrophobe Komponente Isoparaffine enthalten. Dadurch wird jedoch das Haar so stark belastet, daß es "schwer" erscheint, das heißt das Haar zeigt kein oder nur ein geringes Volumen und wenig Halt und Fülle.

In der DE 3009763 werden aus drei Phasen bestehende Mittel beschrieben, welche als hydrophobe Komponenten Paraffine oder Polydimethylsiloxane enthalten. Nach der Anwendung dieses Mittels wird am Haar ebenfalls die zuvor dargestellte Belastung festgestellt.

WO 99/58099 A (SCHWARZKOPF GMBH HANS ;BICHELS DIRK (DE); KNAPPE THORSTEN (DE); MA) 18. November 1999 (1999-11-18) offenbart (Beispiel 4) eine für die dauerhafte Verformung von Keratinfasern geeignete Zusammensetzung, die als Zwei- oder Mehrphasensystem vorliegt, enthaltend ein Silikonöl mit Viskosität bis 50.000 cSt (Dow Corning 344).

In keiner der Schriften findet sich jedoch auch nur der geringste Hinweis auf die erfindungsgemäß ausgewählten Silikonöle und/oder die synergistisch wirksame Kombination von zwei oder mehr hydrophoben Komponenten in einer der Phasen.

Erfindungsgemäß geeignete hydrophobe Komponenten sind Kombination von a) mindestens ein Silikonöl mit einer kinematischen Viskosität bis zu 50.000 mm²s⁻¹ bei 25°C mit b) einen Fettsäure- und Fettalkohol ester, ausgewählt aus modifizierten Cocoglyceriden und /oder Dialkylcarbonat, sowie deren Mischungen mit festen Paraffinen und Wachsen. Bevorzugt sind solche hydrophoben Komponenten, deren Löslichkeit in Wasser bei 20 °C kleiner 1 Gew.-%, insbesondere kleiner als 0,1 Gew.-% beträgt. Der Schmelzpunkt der einzelnen Öl- oder Fettkomponenten liegt bevorzugt unterhalb von etwa 40 °C. Ölkomponenten, die bei Raumtemperatur, d. h. unterhalb von 25 °C flüssig sind, können erfindungsgemäß besonders bevorzugt sein. Bei Verwendung mehrerer hydrophober Komponenten sowie ggf. festen Paraffinen und Wachsen ist es in der Regel jedoch auch ausreichend, wenn die Mischung der hydrophoben Komponenten sowie ggf. Paraffine und Wachse diesen Bedingungen genügt.

Erfindungsgemäß einsetzbare modifizierte Cocoglyceride sind beispielsweise das Handelsprodukt Myritol^{®} 331.

Erfindungsgemäß verwendbare hydrophobe Komponenten sind schließlich auch Silikonöle, insbesondere Dialkyl- und Alkylarylsiloxane, wie beispielsweise Dimethylpolysiloxan und Methylphenylpolysiloxan, sowie deren alkoxylierte und quaternierte Analoga sowie cyclische Siloxane. Beispiele für solche Silikone sind die von Dow Corning unter den Bezeichnungen DC 190, DC 200 und DC 1401 vertriebenen Produkte sowie die Handelsprodukte DC 344 und DC 345 von Dow Coming, Q2-7224 (Hersteller: Dow Corning; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80), solange sie eine kinematische Viskosität bis zu 50.000 cSt gemessen bei 25 °C aufweisen. Ganz besonders bevorzugt sind Silikonöle mit kinematischen Viskositäten bis zu 10.000 cSt gemessen bei 25 °C. Die Bestimmung der Viskositäten erfolgt dabei nach der Kugelfallmethode entsprechend der Methode "british standard 188". Vergleichbare Werte werden mit zum "british standard 188" analogen Prüfvorschriften der Hersteller erhalten, beispielsweise der "CTM 0577" der Dow Corning Corporation.

In einer besonderen Ausführungsform werden als hydrophobe Komponente insbesondere cyclische Siloxane wie beispielsweise die Produkte Dow Corning^{®} 344, Dow Corning^{®} 345, Dow Corning^{®} 244, Dow Corning^{®} 245 oder Dow Corning^{®} 246 mit kinematischen Viskositäten von bis zu 10.000 cSt bei 25 °C bestimmt entsprechend den Angaben des Herstellers eingesetzt.

Erfindungsgemäß einsetzbare hydrophobe Komponenten sind schließlich auch Dialkylcarbonate, wie sie in der DE-OS 197 101 54, ausführlich beschrieben werden. Dioctylcarbonate, insbesondere das Di-2-ethylhexylcarbonat und Dioctylcarbonat, sind bevorzugte hydrophobe Komponenten in Rahmen der vorliegenden Erfindung.

Erfindungsgemäß können zusätzlich zu den genannten hydrophoben Komponenten mit Wasser nur begrenzt mischbare Alkohole eingesetzt werden.

Unter "mit Wasser begrenzt mischbar" werden solche Alkohole verstanden, die in Wasser bei 20 °C zu nicht mehr als 10 Gew.-%, bezogen auf die Wassermasse, löslich sind.

In vielen Fällen haben sich Triole und insbesondere Diole als erfindungsgemäß besonders geeignet erwiesen. Erfindungsgemäß einsetzbar sind Alkohole mit 4 bis 20, insbesondere 4 bis 10, Kohlenstoffatomen. Die erfindungsgemäß verwendeten Alkohole können gesättigt oder ungesättigt und linear, verzweigt oder cyclisch sein. Einsetzbar im Sinne der Erfindung sind beispielsweise Butanol-1, Cyclohexanol, Pentanol-1, Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Erfindungsgemäß bevorzugt als Alkohole sind Butanol-1, Cyclohexanol, und Pentanol-1. Insbesondere Butanol-1 und Cyclohexanol sind besonders bevorzugt.

Die erfindungsgemäßen Wellmittel enthalten zwingend die als keratinreduzierende Substanzen bekannten Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Salze der schwefligen Säure. Bevorzugt geeignet sind die Monoethanolammoniumsalze- oder Ammoniumsalze der Thioglykolsäure und/oder der Thiomilchsäure sowie die freien Säuren. Diese werden in den Wellmitteln bevorzugt in Konzentrationen von 0,5 bis 2,0 mol/kg bei einem pH-Wert von 5 bis 12, insbesondere von 7 bis 9,5, eingesetzt. Zur Einstellung dieses pH-Wertes enthalten die erfindungsgemäßen Wellmittel üblicherweise Alkalisierungsmittel wie Ammoniak, Alkali- und Ammonium-carbonate und -hydrogencarbonate oder organische Amine wie Monoethanolamin.

Als weitere Bestandteile sowohl des Wellmittels als auch der Zwischenspülung und/oder des Fixiermittels können die im folgenden beschriebenen Wirksubstanzen im Rahmen der Erfindung besonders vorteilhaft eingesetzt werden.

Hierzu sind zunächst die Polyole zu rechnen. Geeignete Polyole sind beispielsweise Glycerin und Partialglycerinether, 2-Ethyl-1,3-hexandiol, 1,3-Butandiol, 1,4-Butandiol, 1,2-Propandiol, 1,3-Propandiol, Pentandiole, beispielsweise 1,2-Pentandiol, Hexandiole, beispielsweise 1,2-Hexandiol oder 1,6-Hexandiol, Dodekandiol, insbesondere 1,2-Dodekandiol, Neopentylglykol und Ethylenglykol. Insbesondere 2-Ethyl-1,3-hexandiol, 1,2-Propandiol, 1,3-Propandiol und 1,3-Butandiol haben sich als besonders gut geeignet erwiesen. Ganz besonders bevorzugt sind jedoch 2-Ethyl-1,3-hexandiol und 2-Butandiol.

Diese Polyole sind in den erfindungsgemäß verwendeten Wellmitteln bevorzugt in Mengen von 0,1-10, insbesondere 2 -10, Gew.-%, bezogen auf das gesamte Wellmittel, enthalten.

Kohlenhydrate sind weitere erfindungsgemäß vorteilhaft verwendbare Substanzen. Unter Kohlenhydrate sind insbesondere im Sinne der vorliegenden Erfindung zu verstehen:
- Zuckeralkohole, Zuckersäuren, Zuckersäurederivate und Zucker sowie deren Salze,
   - insbesondere Monosaccharide, Disaccharide, Trisaccharide und Oligosaccharide, wobei diese auch in Form von Aldosen, Ketosen und/oder Lactosen, sowie geschützt durch übliche und in der Literatur bekannte -OH - und NH - Schutzgruppen, wie beispielsweise die Triflatgruppe, die Trimethylsilylgruppe oder Acylgruppen sowie weiterhin in Form der Methylether und als Phosphatester, vorliegen können. Als Beispiele seine genannt Sorbit, Inosit, Mannit, Tetrite, Pentite, Hexite, Threit, Erythrit, Adonit, Arabit, Xylit, Dulcit, Erythrose, Threose, Arabinose, Ribose, Xylose, Lyxose, Glucose, Galactose, Mannose, Allose, Altrose, Gulose, Idose, Talose, Fructose, Sorbose, Psicose und Tegatose.
   - Aminodesoxyzucker, Desoxyzucker, Thiozucker, wobei diese auch in Form von Aldosen, Ketosen und/oder Lactosen, sowie geschützt durch übliche und in der Literatur bekannte -OH - und NH - Schutzgruppen, wie beispielsweise die Triflatgruppe, die Trimethylsilylgruppe oder Acylgruppen sowie weiterhin in Form der Methylether und als Phosphatester, vorliegen können. Als Beispiele seien genannt Desoxyribose, Glucosamin, Galaktosamin und Thioglucose.
   - Onsäuren, wie Gluconsäure, Zuckersäure, Mannozuckersäure, Schleimsäure, Glucuronsäure, Mannonsäure, Ketogluconsäure, Neuraminsäure, Muraminsäure und deren Lactone, wie Gluconsäure-γ-lacton.

   Bevorzugt sind hierunter Onsäuren und Monosaccharide mit 3 bis 8 C - Atomen, wie beispielsweise Triosen, Tetrosen, Pentosen, Hexosen, Heptosen und Octosen, wobei diese auch in Form von Aldosen, Ketosen und/oder Lactosen sowie geschützt durch übliche und in der Literatur bekannte -OH - und NH - Schutzgruppen, wie beispielsweise die Triflatgruppe, die Trimethylsilylgruppe oder Acylgruppen sowie weiterhin in Form der Methylether und als Phosphatester, vorliegen können.
   Ganz besonders bevorzugt sind die Onsäuren und deren Lactone und Derivate, insbesondere die Salze der Gluconsäure, wobei die Salze mit den Alkalimetallen, Erdalkalimetallen, den Elementen der Gruppen IB, IIB, VIIIB des Periodensystemes - gemäß der IUPAC - Empfehlung von 1985 - sowie die Ammoniumsalze bevorzugt sind. Hierunter wiederum sind ganz besonders bevorzugt sind die Salze mit den Elementen der IB - Gruppe, insbesondere Kupfer.

Weiterhin sei auf die einschlägige Fachliteratur wie beispielsweise Beyer-Walter, Lehrbuch der organischen Chemie, S. Hirzel Verlag Stuttgart, 19. Auflage, Abschnitt III Seiten 393 und folgende verwiesen.

Selbstverständlich umfaßt die erfindungsgemäße Lehre alle isomeren Formen, wie cis - trans - Isomere, Diastereomere, Epimere, Anomere und chirale Isomere.

Erfindungsgemäß ist es auch möglich, eine Mischung aus mehreren Kohlenhydraten einzusetzen.

Die erfindungsgemäßen Kohlenhydrate sind in den Mitteln in Konzentrationen von 0,005 Gew.% bis zu 20 Gew.%, vorzugsweise von 0,005 Gew.% bis zu 10 Gew.% und ganz besonders bevorzugt in Mengen von 0,1 Gew.% bis zu 5 Gew.% enthalten.

Weiterhin hat es sich gezeigt, daß vorteilhafterweise Polymere (G) im Rahmen des erfindungsgemäßen Verfahrens eingesetzt werden. In einer bevorzugten Ausführungsform werden den erfindungsgemäß verwendeten Mitteln daher Polymere zugesetzt, wobei sich sowohl kationische, anionische, amphotere als auch nichtionische Polymere als wirksam erwiesen haben.

Unter kationischen Polymeren (G1) sind Polymere zu verstehen, welche in der Haupt-und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 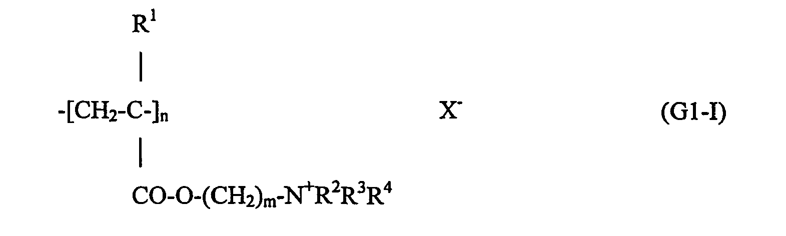 in der R¹= -H oder -CH₃ ist, R², R³ und R⁴ unabhängig voneinander ausgewählt sind aus C1-4-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylen-bisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vemetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JI^{®} im Handel erhältlich sind. Die Verbindungen Celquat^{®} H 100, Celqua^{®} L 200 und Polymer JR^{®} 400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Coming; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquatemäre Polydimethylsiloxane, Quatemium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Bei den anionischen Polymeren (G2), welche in den Zubereitungen des erfindungsgemäßen Verfahrens verwendet werden können, handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Beispielsweise ist ein solches Homopolymer der 2-Acrylamido-2-methylpropansulfonsäure, unter der Bezeichnung Rheothik^{®}11-80 im Handel erhältlich.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.
Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vemetzungsagentien bevorzugt polyolefinisch ungesättigte Verbindungen wie Tetraallyloxyethan, Allylsucrose, Allylpentaerythrit und Methylen-bisacrylamid zum Einsatz kommen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls gut geeignete Polymere. Ein mit 1,9-Decadiene vernetztes Maleinsäure-Methylvinylether-Copolymer ist unter der Bezeichnungg Stabileze^{®} QM im Handel erhältlich.

Weiterhin können als Polymere in allen wäßrigen Zubereitungen des erfindungsgemäßen Verfahrens amphotere Polymere (G3) verwendet werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppe und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (G3-I),

   R¹-CH=CR²-CO-Z-(CₙH₂ₙ)-N⁽⁺⁾R³R⁴R⁵ A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (G3-II),

   R⁶-CH=CR⁷-COOH (G3-II)
in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate : deutsche Offenlegungsschrift 39 29 973. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Weiterhin können in allen wäßrigen Zubereitungen des erfindungsgemäßen Verfahrens nichtionogene Polymere (G4) enthalten sein. Es kann bevorzugt sein, diese in der Fixierung (C) und /oder deren Komponente (C2) zu verwenden.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack
- Polyvinylpyrrolidone, wie sie beispielsweise unter der Bezeichnung Luviskol^{®} (BASF) vertrieben werden.
- Glycosidisch substituierte Silicone gemäß der EP 0612759 B1.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Die Polymere (G) sind in den erfindungsgemäß verwendeten Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Weiterhin können in den erfindungsgemäß verwendeten Zubereitungen Proteinhydrolysate und/oder Aminosäuren und deren Derivate (H) enthalten sein. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 5000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen und/oder marinen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda), Crotein^{®} (Croda), Promois^{®} (Seiwa) oder Sea Silk^{®} (Rovi) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda) oder Crotein^{®} (Croda) vertrieben.

Die Proteinhydrolysate oder deren Derivate sind in den erfindungsgemäß verwendeten Zubereitungen bevorzugt in Mengen von 0,01 bis 10 Gew.-%, bezogen auf das gesamte Mittel, enthalten. Mengen von 0,01 bis 5 Gew.-% sind besonders bevorzugt.

Weiterhin können 2-Pyrrolidinon-5-carbonsäure und/oder deren Derivate (J) in den Zubereitungen des erfindungsgemäßen Verfahrens verwendet werden. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Ebenfalls als vorteilhaft hat sich die Verwendung von Vitaminen, Provitaminen und Vitaminvorstufen sowie deren Derivaten (K) erwiesen.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1-5 Gew.% sind besonders bevorzugt.
- Vitamin B₆ (Pyridoxin sowie Pyridoxamin und Pyridoxal),
- Vitamin C (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.
- Vitamin F. Unter dem Begriff "Vitamin F" werden üblicherweise essentielle Fettsäuren, insbesondere Linolsäure, Linolensäure und Arachidonsäure, verstanden.
- Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäß verwendeten Zubereitungen Vitamine, Provitamine und Vitaminvorstufen aus den Gruppen A, B, E und H.

Panthenol, Pantolacton, Pyridoxin und seine Derivate sowie Nicotinsäureamid und Biotin sind besonders bevorzugt.

Schließlich können in den Zubereitungen des erfindungsgemäßen Verfahrens Pflanzenextrakten (L) verwendet werden.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren bevorzugt in der Pflegekomponente (B) und/oder der Fixierung (C) einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8-22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Weitere Bestandteile aller wäßrigen Zubereitungen können erfindungsgemäß oberflächenaktive Verbindungen sein, insbesondere solche aus der Gruppe der anionischen, amphoteren, zwitterionischen und/oder nichtionischen Tenside (E).

Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wäßriger Lösung stark hydratisiert sind. Weitergehende Definitionen und Eigenschaften von Tensiden finden sich in "H.-D.Dörfler, Grenzflächen- und Kolloidchemie, VCH Verlagsgesellschaft mbH. Weinheim, 1994". Die zuvor wiedergegebene Begriffsbestimmung findet sich ab S. 190 in dieser Druckschrift.

Als anionische Tenside (E1) eignen sich in erfindungsgemäßen Zubereitungen prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 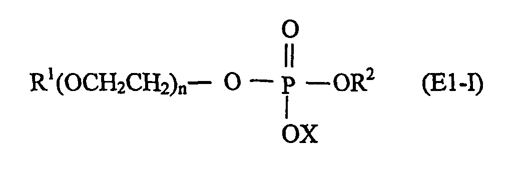 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (E1-III) 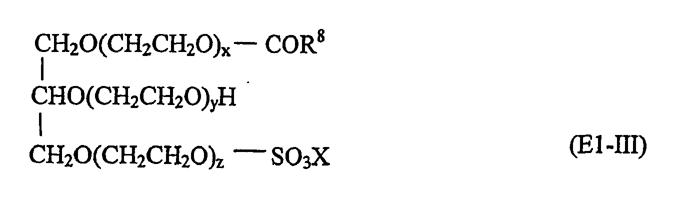 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht, wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind,
- Amidethercarbonsäuren wie sie in der EP 0 690 044 beschrieben sind,
- Kondensationsprodukte aus C₈ - C₃₀ - Fettalkoholen mit Proteinhydrolysaten und/oder Aminosäuren und deren Derivaten,welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl-und Alkenyletherphosphate sowie Eiweissfettsäurekondensate.

Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (E3) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Grupp enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993), B. Salka in Cosm.Toil. 108, 89 (1993) sowie J. Kahre et al. in SÖFW-Journal Heft 8, 598 (1995) verwiesen.
   Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III), 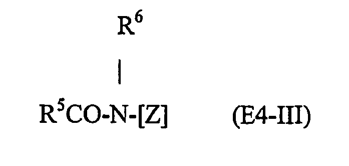 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften US 1,985,424, US 2,016,962 und US 2,703,798 sowie die Internationale Patentanmeldung WO 92/06984 verwiesen. Eine Übersicht zu diesem Thema von H.Kelkenberg findet sich in Tens. Surf. Det. 25, 8 (1988). Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄CH₂OH (E4-IV)

   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin sind ganz besonders bevorzugte nichtionische Tenside die Zuckertenside. Diese können in den erfindungsgemäß verwendeten Mitteln bevorzugt in Mengen von 0,1 - 20 Gew.-%, bezogen auf das gesamte Mittel, enthalten sein. Mengen von 0,5-15 Gew.-% sind bevorzugt, und ganz besonders bevorzugt sind Mengen von 0,5 - 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside (E) werden in Mengen von 0,1 - 45 Gew.%, bevorzugt 0,5 - 30 Gew.% und ganz besonders bevorzugt von 0,5 - 25 Gew.%, bezogen auf das gesamte erfindungsgemäß verwendete Mittel, eingesetzt.

In einer weiteren bevorzugten Ausführungsform werden in den Zubereitungen des erfindungsgemäßen Verfahrens Emulgatoren (F) verwendet. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D.Dörfler, Grenzflächen- und Kolloidchemie, VCH Verlagsgesellschaft mbH. Weinheim, 1994". Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®} 68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,1-3 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.
Als weitere Pflegestoffe können in der wäßrigen Pflegekomponente (B) heterocyclische Verbindungen wie beispielsweise Derivate von Imidazol, Pyrrolidin, Piperidin, Dioxolan, Dioxan, Morpholin und Piperazin eingesetzt werden. Weiterhin eignen sich Derivate dieser Verbindungen wie beispielsweise die C₁₋₄-Alkyl-Derivate, C₁₋₄-Hydroxyalkyl-Derivate und C₁₋₄-Aminoalkyl-Derivate. Bevorzugte Substituenten, die sowohl an Kohlenstoffatomen als auch an Stickstoffatomen der heterocyclischen Ringsysteme positioniert sein können, sind Methyl-, Ethyl-, β-Hydroxyethyl- und β-Aminoethyl-Gruppen.

Bevorzugt enthalten diese Derivate 1 oder 2 dieser Substituenten.

Erfindungsgemäß bevorzugte Derivate heterocyclischer Verbindungen sind beispielsweise 1-Methylimidazol, 2-Methylimidazol, 4(5)-Methylimidazol, 1,2-Dimethylimidazol, 2-Ethylimidazol, 2-Isopropylimidazol, N-Methylpyrrolidon, 1-Methylpiperidin, 4-Methylpiperidin, 2-Ethylpiperidin, 4-Methylmorpholin, 4-(2-Hydroxyethyl)morpholin, 1-Ethylpiperazin, 1-(2-Hydroxyethyl)piperazin, 1-(2-Aminoethyl)piperazin. Weiterhin erfindungsgemäß bevorzugte Imidazolderivate sind Biotin, Hydantoin und Benzimidazol.

Unter diesen heterocyclischen Pflegestoffen sind die Mono- und Dialkylimidazole, Biotin und Hydantoin besonders bevorzugt.

Diese heterocyclischen Verbindungen sind in den erfindungsgemäßen Wellmitteln in Mengen von 0,5 bis 10 Gewichts-%, bezogen auf das gesamte Wellmittel, enthalten. Mengen von 2 bis 6 Gew.% haben sich als besonders geeignet erwiesen.

Weitere Pflegesubstanzen, welche in allen erfindungsgemäß verwendeten wäßrigen Zubereitungen enthalten sein können, sind erfindungsgemäß Aminosäuren und Aminosäurederivate. Aus der Gruppe der Aminosäuren haben sich insbesondere Arginin, Asparagin, Asparaginsäure, Citrullin, Histidin, Ornithin und Lysin als erfindungsgemäß geeignet erwiesen. Die Aminosäuren können sowohl als freie Aminosäure, als auch als Salze, z. B. als Hydrochloride oder der Alkali, Erdalkali oder Ammoniumsalze eingesetzt werden. Weiterhin haben sich auch Oligopeptide aus durchschnittlich 2-3 Aminosäuren, die einen hohen Anteil (> 50 %, insbesondere > 70 %) an den genannten Aminosäuren haben, als erfindungsgemäß einsetzbar erwiesen.

Erfindungsgemäß besonders bevorzugt sind Arginin, Asparagin, Asparaginsäure sowie deren Salze und Oligopeptide bzw. Hydrolysate, welche reich an den genannten bevorzugten Aminsöuren sind. Ganz besonders bevorzugt sind Asparagin und Asparaginsäure sowie deren Salze bzw. Hydrolysate.

Diese Aminosäuren bzw. Derivate sind in den erfindungsgemäßen Zubereitungen in Mengen von 0,001 bis 10 Gewichts-%, bezogen auf das gesamte Mittel, enthalten. Mengen von 0,001 bis 3 Gew.-% haben sich als besonders geeignet erwiesen.

Zusätzlich kann es sich als vorteilhaft erweisen, wenn die erfindungsgemäßen Wellmittel Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Die Penetrationshilfsstoffe und Quellmittel sind in den erfindungsgemäß eingesetzten Zubereitungen in Mengen von 0,1 bis 20 Gewichts-%, bezogen auf das gesamte Mittel, enthalten. Mengen von 01, bis 10 Gewichts-% sind bevorzugt.

Weiterhin können die erfindungsgemäßen Wellmittel wellkraftverstärkende Komponenten, insbesondere Harnstoff, Imidazol und die oben genannten Diole enthalten. Bezüglich näherer Informationen zu solchen wellkraftverstärkenden Komponenten: Druckschriften DE-OS 44 36 065 und EP-B1-363 057.

Die wellkraftverstärkenden Verbindungen können in den erfindungsgemäßen Wellmitteln in Mengen von 0,5 bis 5 Gew.-%, bezogen auf das gesamte Wellmittel, enthalten sein. Mengen von 1 bis 4 Gew.-% haben sich als ausreichend erwiesen, weshalb diese Mengen besonders bevorzugt sind.

Zwingender Bestandteil der erfindungsgemäßen Fixiermittel sind Oxidationsmittel, z.B. Natriumbromat, Kaliumbromat, Wasserstoffperoxid und die zur Stabilisierung wäßriger Wasserstoffperoxidzubereitungen üblichen Stabilisatoren. Der pH-Wert solcher wäßriger H₂O₂-Zubereitungen, die üblicherweise etwa 0,5 bis 15 Gew.-%, gebrauchsfertig in der Regel etwa 0,5 - 3 Gew.-% H₂O₂ enthalten, liegt bevorzugt bei 2 bis 6, insbesondere 2 bis 4; er wird durch Säuren, bevorzugt Phosphorsäure, Phosphonsäuren und/oder Dipicolinsäure, eingestellt. Im Rahmen des erfindungsgemäßen Verfahrens ist es möglich und kann es bevorzugt sein, die erforderliche H₂O₂-Menge mittels eines größeren Volumens der fixierenden Zubereitung mit vergleichsweise niedrigeren H₂O₂-Konzentrationen, insbesondere im Bereich von 0,5-1,5 Gew.-%, einzusetzen. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Erfindungsgemäß besonders bevorzugt kann die Verwendung von Fixiermittel-Konzentraten sein, die vor Anwendung mit Wasser verdünnt werden.

Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel, oder auch Enzyme verwendet werden, die Elektronen aus geeigneten Entwicklerkomponenten (Reduktionsmittel) auf Luftsauerstoff übertragen. Bevorzugt sind dabei Oxidasen wie Tyrosinase, Ascorbatoxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Die erfindungsgemäßen Fixiermittel können selbstverständlich auch als Feststoffe formuliert werden. Sie enthalten das Oxidationsmittel dann in Form eines Festkörpers, z.B. Kalium- oder Natriumbromat. Erst kurz vor der Anwendung werden diese Mittel dann mit Wasser und/oder einer weiteren Komponente versetzt. Ebenfalls möglich und bevorzugt ist, das Oxidationsmittel als 2-Komponenten-System zu formulieren. Die beiden Komponenten, von denen die eine bevorzugt eine Wasserstoffperoxidlösung oder eine wäßrige Lösung eines anderen Oxidationsmittels ist und die andere die übrigen Bestandteile, insbesondere pflegende Substanzen und/oder Reduktionsmittel, enthält, werden ebenfalls erst kurz vor der Anwendung vermischt.

Neben den im Zusammenhang mit dem Wellmittel genannten Komponenten können die Fixiermittel als oberflächenaktive Verbindungen kationische Tenside (E5) vom Typ der quarternären Ammoniumverbindungen, der Esterquats und der Amidoamine enthalten. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside (E5) sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie
- quaternierte Amine wie Methyl-1-alkylamidoethyl-2-alkylimidazolinium-methosulfat,
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Antioxidantien.

Bezüglich der weiteren Bestandteile der erfindungsgemäßen Mittel und deren übliche Einsatzmengen wird ausdrücklich auf die bekannten Monographien, z. B. Umbach, Kosmetik, 2. Auflage, Georg Thieme Verlag, Stuttgart New York, 1995, und Kh. Schrader, Grundlagen und Rezepturen der Kosmetika, 2. Auflage, Hüthig Buch Verlag, Heidelberg, 1989, verwiesen.

Die wäßrigen und die nichtwäßrigen Phasen liegen in den in dem erfindungsgemäßen Verfahren verwendeten Mitteln in Mengenverhältnissen (bezogen auf das Gewicht) von 1:200 bis 1:1, bevorzugt von 1:40 bis 1:5 und besonders bevorzugt von 1:20 bis 1:10 vor. In Fällen, in denen mehrere nichtwäßrige Phasen vorliegen, beziehen sich diese Angaben auf die Gesamtheit der nichtwäßrigen Phasen.

Die erfindungsgemäße Lehre umfaßt auch solche Ausführungsformen des erfindungsgemäßen Verfahrens, bei denen das mehrphasige Mittel aus zwei oder mehr getrennt konfektionierten Ausgangszubereitungen erst unmittelbar vor der Anwendung hergestellt wird. Diese Ausführungsform kann bei extrem inkompatiblen Bestandteilen bevorzugt sein.

Ein weiterer Gegenstand der Anmeldung sind die zur Durchführung des erfindungsgemäßen Verfahrens verwendeten Mittel.

Diese Mittel dienen im Rahmen eines Verfahrens zur dauerhaften Verformung von Keratinfasern entweder zur Durchführung der reduzierenden Stufe, zur Durchführung der oxidierenden Stufe oder zum Spülen nach Durchführung der reduzierenden Stufe und können prinzipiell alle für diese Mittel üblichen Bestandteile enthalten, sofern die erfindungsgemäßen Bedingungen (Vorliegen des Zwei- oder Mehrphasensystems und die kurzzeitige Mischbarkeit) gegeben sind.

Bevorzugt wird das erfindungsgemäße Verfahren zum Dauerwellen bzw. Glätten von menschlichen Haaren verwendet.

Ein zweiter Gegenstand der Erfindung ist daher ein Mittel gemäß Anspruch 4 zur Durchführung der reduzierenden Stufe eines Verfahrens zur dauerhaften Verformung von Keratinfasern enthaltend eine keratinreduzierende Substanz sowie übliche Bestandteile, dadurch gekennzeichnet, daß es in Form eines Zwei- oder Mehrphasensystems vorliegt, das mindestens eine hydrophobe Komponente enthält, welche zusammengesetzt ist aus
a. mindestens einem Silikonöl mit einer kinematischen Viskosität bis zu 50.000 cSt bei 25 °C und
b. Fettsäure- und Fettalkoholestern ausgewählt aus modifizierten Cocoglyceriden und/oder Dialkylcarbonaten, wobei die Gewichtsverhältnisse von a. zu b. 100:1 bis 1:1 betragen,
und das durch mechanische Bewegung in ein homogenes System überführt werden kann.

Ein dritter Gegenstand der Erfindung ist ein Mittel zur Durchführung der oxidierenden Stufe eines Verfahrens zur dauerhaften Verformung von Keratinfasern enthaltend ein Oxidationsmittel sowie übliche Bestandteile, dadurch gekennzeichnet, daß es in Form eines Zwei- oder Mehrphasensystems vorliegt, das mindestens eine hydrophobe Komponente enthält, welche zusammengesetzt ist aus
a. mindestens einem Silikonöl mit einer kinematischen Viskosität bis zu 50.000 cSt bei 25 °C und
b. Fettsäure- und Fettalkoholestern ausgewählt aus modifizierten Cocoglyceriden und/oder Dialkylcarbonaten, wobei die Gewichtsverhältnisse von a. zu b. 100:1 bis 1:1 betragen,
und das durch mechanische Bewegung in ein homogenes System überführt werden kann.

Ein vierter Gegenstand der Erfindung ist ein Mittel zum Spülen nach Durchführung der reduzierenden Stufe eines Verfahrens zur dauerhaften Verformung von Keratinfasern enthaltend übliche Bestandteile, dadurch gekennzeichnet, daß es in Form eines Zwei-oder Mehrphasensystems vorliegt, das mindestens eine hydrophobe Komponente enthält, welche zusammengesetzt ist aus
a. mindestens einem Silikonöl mit einer kinematischen Viskosität bis zu 50.000 cSt bei 25 °C und
b. Fettsäure- und Fettalkoholestern ausgewählt aus modifizierten Cocoglyceriden und/oder Dialkylcarbonaten, wobei die Gewichtsverhältnisse von a. zu b. 100:1 bis 1:1 betragen,
und das durch mechanische Bewegung in ein homogenes System überführt werden kann.

Die folgenden Beispiele sollen die Erfmdung näher erläutern.

### Beispiele

Soweit nicht anders vermerkt, sind alle Angaben Gewichtsteile

| **Phase 1** | **B1** | **B2** | **V1** | **V2** | **B3** | **B4** | **V3** |
|---|---|---|---|---|---|---|---|
| Ammoniumthioglykolat (71% | 18,20 | 18,20 | 18,20 | 18,20 | 12,00 | 12,00 | 12,00 |
| in Wasser) | | | | | | | |
| Ammoniumthiolactat (70% in | 1,30 | 1,30 | 1,30 | 1,30 | --- | --- | --- |
| Wasser) | | | | | | | |
| Harnstoff | 2,10 | 2,10 | 2,10 | 2,10 | --- | --- | --- |
| Turpinal^{®1} SL | 0,30 | 0,30 | 0,30 | 0,30 | 0,30 | 0,30 | 0,30 |
| Propylenglykol-1,2 | 2,10 | 2,10 | 2,10 | 2,10 | 2,10 | 2,10 | 2,10 |
| Ammoniak (25% in Wasser) | 2,60 | 2,60 | 2,60 | 2,60 | 1,60 | 1,60 | 1,60 |
| Ammoniumhydrogencarbonat | 6,00 | 6,00 | 6,00 | 6,00 | 6,00 | 6,00 | 6,00 |
| Promois Silk^{®2} | 0,5 | 0,06 | 0,06 | 0,06 | 0,21 | 0,21 | 0,21 |
| Merquat^{®3}100 | 0,06 | 0,06 | 0,06 | 0,06 | 0,21 | 0,21 | 0,21 |
| Lamepon^{®4} S | 0,53 | 0,53 | 0,53 | 0,53 | 0,53 | 0,53 | 0,53 |
| Wasser | ad 100,00 | ad 100 | ad 100,00 | ad 100,00 | ad 100,00 | ad 100,00 | ad 100,00 |

| | | | | | | | |
|---|---|---|---|---|---|---|---|
| ¹Organische Phosphonsäure (INCI - Bezeichnung: Etidronic Acid), Aktivgehalt ca. 58 - 61% (COGNIS) ² Seidenproteinhydrolysat (INCI - Bezeichnung: Hydrolyzed Silk) (SEIWA KASEI) ³ Poly(dimethyldiallylammoniumchlorid) (40 % Aktivsubstanz; INCI - Bezeichnung: Polyquaternium-6) (CHEMVIRON) ⁴Protein-Kokosfettsäurekondensat, Kaliumsalz (INCI - Bezeichnung: Potassium Cocoyl Hydrolyzed Collagen) Aktivgehalt ca. 32% (COGNIS) | | | | | | | |

| **Phase 2** | **B1** | **B2** | **V1** | **V2** | **B3** | **B4** | **V3** |
|---|---|---|---|---|---|---|---|
| Myritol^{®5} 331 | 10,0000 | --- | --- | --- | 10,0000 | --- | --- |
| Cetiol^{®6} CC | --- | 10,0000 | --- | --- | --- | --- | --- |
| Parfüm | 10,0000 | 10,0000 | 10,0000 | 10,0000 | 10,0000 | 10,0000 | 10,0000 |
| C.I. 12700 | 0,0005 | 0,0005 | 0,0005 | 0,0005 | 0,0005 | 0,0005 | 0,0005 |
| C.I.61565 | 0,0008 | 0,0008 | 0,0008 | 0,0008 | 0,0008 | 0,0008 | 0,0008 |
| Dow Corning 345^{®7} | ad 100,0000 | ad 100,0000 | --- | ad 100,0000 | ad 100,0000 | --- | --- |
| Dow Corning 200 Fluid^{®8} 100 cSt | --- | --- | --- | --- | --- | ad 100,0000 | --- |
| Dow Corning 200 Fluid^{®9} 60.000 cSt | --- | --- | --- | --- | --- | --- | ad 100,0000 |
| Paraffinöl | --- | --- | ad 100,0000 | --- | --- | --- | --- |

| | | | | | | | |
|---|---|---|---|---|---|---|---|
| ⁵ modifiziertes Kokosöl (INCI- Bezeichnung: Cocoglycerides) (COGNIS) ⁶ INCI - Bezeichnung: Dicaprylyl Carbonate (COGNIS) ⁷ INCI - Bezeichnung: Cyclomethicone (DOW CORNING) ⁸ INCI - Bezeichnung: Cyclomethicone (Dow Coming) ⁹ INCI - Bezeichnung: Dimethicone (Dow Coming) | | | | | | | |

### Ergebnisse zur Anwendung der Beispiele 1 bis 3:

Unmittelbar vor der Anwendung werden in diesen Beispielen jeweils 75 ml der Phase 1 mit jeweils 5 ml der Phase 2 gemischt.

Beispiel 1 und 2 sind Dauerwellen für normales Haar.
Die Formulierungen B1 und B2 sind für die Anwendung ausreichend lange stabile 2-Phasen Wellotionen. Nach der Anwendung zeigt das gewellte Haar eine sehr gute Sprungkraft und hervorragende Pflegeleistung.
Die nicht erfindungsgemäße Vergleichsformulierung V1 entsprechend dem Stand der Technik hinterläßt das Haar nach der Anwendung stark belastet mit geringer Sprungkraft der Locken.
Die nicht erfindungsgemäße Vergleichsformulierung 2 ist bereits ästhetisch nicht ansprechend, weil die Farbstoffe zur Anfärbung nicht gelöst sind. Weiterhin ist diese Formulierung nach dem Mischen der beiden Phasen nicht ausreichend lange homogen.

Beispiel 3 ist eine Dauerwelle für gefärbtes Haar.
Die Formulierung B3 ansprechend gefärbt. Nach der Anwendung zeigt das Haar eine sehr gute Sprungkraft und wirkt sehr gut gepflegt.

Beispiel 4 ist wiederum eine Dauerwelle für gefärbtes Haar.
Nach der Anwendung zeigt sich das Haar hervorragend gepflegt mit sehr guter Sprungkraft.
Die nicht erfindungsgemäße Vergleichsformulierung wie sie beispielsweise dem Stand der Technik der DE 3009763 entspricht, hinterläßt das Haar deutlich belastet mit nur geringer Sprungkraft.

### Beispiel 5 (nicht im Schutzbereich)

### Komponente A

| Phase 1 | |
|---|---|
| Ammoniak 25% | 0,88 |
| Ammoniumhydrogencarbonat | 5,49 |
| Merquat^{®} 100 | 0,10 |
| Magnesiumaspartat | 0,01 |
| Kupfergluconat | 0,01 |
| Wasser | ad 100,00 |

### Phase 2

| | |
|---|---|
| Parfüm | 10,000 |
| C.I. 61565 | 0,002 |
| Dow Corning^{®} 345 | ad 100,000 |

### Komponente B

| | |
|---|---|
| Ammoniumthioglykolat (61 % in | 57,3 |
| Wasser) | |
| Ammoniumthiolactat (70% TMS) | 21,4 |
| Turpinal^{®} SL | 1,0 |
| Eumulgin^{®10} L | 4,0 |
| Wasser | ad 100,0 |

| | |
|---|---|
| ¹⁰ 2-Hydroxyfettalkoholalkoxylat, INCI - Bezeichnung: PPG-2-Ceteareth-9 (COGNIS) | |

Die zweiphasige Komponente A ist ansprechend gefärbt. Nach Vermischen von 52,5 ml der Phase 1 mit 5,0 ml der Phase 2 wird die Komponente A erhalten. Aus dieser und 22,5 ml der Komponente B ergibt sich eine anwendungsfertige, ebenfalls ästhetische ansprechende Wellotion. Nach Anwendung im Rahmen einer üblichen Wellbehandlung wurde eine Welle mit sehr guter Sprungkraft und hervorragend gepflegtem Haar erhalten.

### Beispiel 6: Zwischenspülung

| | |
|---|---|
| Dioctylcarbonat | 5,0 |
| Dow Corning^{®} 345 | 5,0 |
| Paraffinöl | 2,0 |
| Wasser | 93,0 |

Durch Anwendung der Zwischenspülung war das Haar nach der Behandlung gut kämmbar und wirkte sehr gepflegt.

### Beispiel 7 Fixiermittel (3 Phasen)

| | |
|---|---|
| Wasserstoffperoxid (50 %ig in Wasser) | 4,0 |
| Aromox^{®}MCD-W¹¹ | 1,0 |
| Turpinal^{®}SL | 1,0 |
| Dioctylcarbonat | 5,0 |
| Dow Corning^{®} 200 Fluid 100cS | 2,0 |
| Paraffinöl | 2,0 |
| Wasser | 82,0 |

| | |
|---|---|
| ¹¹ N,N-Dimethyl-N-kokosalkylamin-N-oxid (30 % Aktivsubstanz in Wasser; INCI-Bezeichnung: Cocamine Oxide (AKZO) | |

Mit Hilfe der erfindungsgemäßen Fixierung war das Haar nach der Behandlung sehr gut kämmbar und wirkte sehr gepflegt.

### Beispiel 8: (nicht im Schutzbereich)

### Komponente A

### Phase 1

| | |
|---|---|
| Ammoniak 25% | 0,88 |
| Ammoniumhydrogencarbonat | 5,49 |
| Merquat^{®} 100 | 0,20 |
| Sea Silk^{®12} | 1,50 |
| Wasser | ad 100,00 |

| | |
|---|---|
| ¹² INCI-Bezeichnung: Aqua AND Himanthalia Elongata AND Enteromorpha Compressa (ROVI GmbH) | |

### Phase 2

| | |
|---|---|
| Parfüm | 10,000 |
| C.I. 61565 | 0,002 |
| Dow Corning^{®} 345 | Ad 100,000 |

### Komponente B

| | |
|---|---|
| Ammoniumthioglykolat (61 % in | 57,3 |
| Wasser) | |
| Ammoniumthiolactat (70% TMS) | 21,4 |
| Turpinal^{®} SL | 1,0 |
| Eumulgin^{®} L | 4,0 |
| Wasser | ad 100,0 |

Die zweiphasige Komponente A ist ansprechend gefärbt. Nach Vermischen von 52,5 ml der Phase 1 mit 5,0 ml der Phase 2 wird die Komponente A erhalten. Aus dieser und 22,5 ml der Komponente B ergibt sich eine anwendungsfertige, ebenfalls ästhetische ansprechende Wellotion. Nach Anwendung im Rahmen einer üblichen Wellbehandlung wurde eine Welle mit sehr guter Sprungkraft und hervorragend gepflegtem Haar erhalten.

### Beispiel 9: Zweiphasenwellmittel: (nicht im Schutzbereich)

### Phase 1

| | |
|---|---|
| Ammoniumthioglykolat (71% in | 18,0 |
| Wasser) | |
| Ammoniak 25% | 3,5 |
| Ammoniumbicarbonat | 9,0 |
| Merquat^{®} 100 | 0,2 |
| Turpinal^{®} SL | 0,3 |
| Protelan^{®}13 VE/K | 1,5 |
| Wasser | ad 100,0 |

### Phase 2

| | |
|---|---|
| Parfüm | 5,0 |
| Tegobetain^{®14} HS | 5,0 |
| Dow Corning^{®} 345 | 20,0 |
| Wacker Belsil^{®15} ADM 652 | 8,0 |
| Wasser | ad 100 |

| | |
|---|---|
| ¹³ INCI-Bezeichnung: Sodium Cocoyl Hydrolyzed Wheat Protein, ca 25% Aktivsubstanz in Wasser (Zschimmer & Schwarz) ¹⁴ NCI-Bezeichnung: Cocamidopropyl Betaine AND Glyceryl Laurate (Goldschmidt) ¹⁵ INCI-Bezeichnung: Amodimethicone (Wacker) | |

### Beispiel 10:Zweiphasenwellmittel: (nicht in Schutzbereich)

Phase 1

| | |
|---|---|
| Ammoniumthioglykolat (71% in | 18,0 |
| Wasser) | |
| Ammoniak 25% | 3,5 |
| Harnstoff | 3,0 |
| Ammoniumbicarbonat | 9,0 |
| Merquat^{®} 100 | 0,2 |
| Turpinal^{®} SL | 0,3 |
| Protelan^{®} VE/K | 1,5 |
| Wasser | ad 100,0 |

### Phase 2

| | |
|---|---|
| Parfüm | 5,0 |
| Tegobetain^{®} HS | 5,0 |
| Dow Corning^{®} 345 | 20,0 |
| Wacker Belsil^{®} ADM 652 | 8,0 |
| Wasser | ad 100,0 |

## Patentansprüche

1. Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, gegebenenfalls nach einer Einwirkungszeit mit einer ersten Spülung spült, dann mit einer wäßrigen Zubereitung eines Oxidationsmittels fixiert und ebenfalls nach einer Einwirkungszeit spült und/oder gegebenenfalls nachbehandelt, **dadurch gekennzeichnet, daß** mindestens eine der beiden wäßrigen Zubereitungen oder die erste Spülung in Form eines Zwei-oder Mehrphasensystems vorliegt, das mindestens eine hydrophobe Komponente enthält, und das zum Aufbringen auf die Faser durch mechanische Bewegung in ein homogenes System überführt wird, wobei die hydrophobe Komponente
a. mindestens ein Silikonöl mit einer kinematischen Viskosität bis zu 50.000 mm²s⁻¹ (50.000 cSt) bei 25 °C und
b. einen Fettsäure- und Fettalkoholester, ausgewählt aus modifizierten Cocoglyceriden und/oder ein Dialkylcarbonat
enthält, wobei die Gewichtsverhältnisse von a. zu b. 100:1 1 bis 1:1 betragen.

2. Verfahren zur dauerhaften Verformung von Keratinfasern nach Anspruch 1, **dadurch gekennzeichnet, daß** die hydrophobe Komponente weiterhin mindestens einen mit Wasser nur begrenzt mischbaren Alkohol enthält.

3. Verfahren zur dauerhaften Verformung von Keratinfasern nach Anspruch 1, **dadurch gekennzeichnet, daß** die hydrophobe Komponente weiterhin mindestens ein Kohlenhydrat enthält.

4. Mittel zur Durchführung der reduzierenden Stufe eines Verfahrens zur dauerhaften Verformung von Keratinfasern enthaltend eine Eceratinreduzierende Substanz sowie übliche Bestandteile, **dadurch gekennzeichnet, daß** es in Form eines Zwei- oder Mehrphasensystems vorliegt, das mindestens eine hydrophobe Komponente enthält, welche zusammengesetzt ist aus
a. mindestens einem Silikonöl mit einer kinematischen Viskosität bis zu 50.000 mm²s⁻¹ (50.000 cSt) bei 25 °C und
b. einen Fettsäure- und Fettalkoholester, ausgewählt aus modifizierten Cocoglyceriden und/oder ein Dialkylcarbonat, wobei die Gewichtsverhältnisse von a. zu b. 100:1 bis 1:1 betragen,
und das durch mechanische Bewegung in ein homogenes System überführt werden kann.

5. Mittel zur Durchführung der oxidierenden Stufe eines Verfahrens zur dauerhaften Verformung von Keratinfasern enthaltend ein Oxidationsmittel sowie übliche Bestandteile, **dadurch gekennzeichnet, daß** es in Form eines Zwei- oder Mehrphasensystems vorliegt, das mindestens eine hydrophobe Komponente enthält, welche zusammengesetzt ist aus
a. mindestens einem Silikonöl mit einer kinematischen Viskosität bis zu 50.000 mm²s⁻¹ (50.000 cSt) bei 25 °C und
b. einen Fettsäure- und Fettalkoholester, ausgewählt aus modifizierten Cocoglyceriden und/oder ein Dialkylcarbonat, wobei die Gewichtsverhältnisse von a. zu b. 100:1 1 bis 1:1 betragen,
und das durch mechanische Bewegung in ein homogenes System überführt werden kann.

6. Mittel zum Spülen nach Durchführung der reduzierenden Stufe eines Verfahrens zur dauerhaften Verformung von Keratinfasern enthaltend übliche Bestandteile, **dadurch gekennzeichnet, daß** es in Form eines Zwei- oder Mehrphasensystems vorliegt, das mindestens eine hydrophobe Komponente enthält, welche zusammengesetzt ist aus
a. mindestens einem Silikonöl mit einer kinematischen Viskosität bis zu 50.000 mm²s⁻¹ (50.000 cSt) bei 25 °C und
b. einen Fettsäure- und Fettalkoholester, ausgewählt aus modifizierten Cocoglyceriden und/oder ein Dialkylcarbonat, wobei die Gewichtsverhältnisse von a. zu b. 100:1 bis 1:1 betragen,
und das durch mechanische Bewegung in ein homogenes System überführt werden kann.

7. Mittel nach einem der Ansprüche 4 bis 6, **dadurch gekennzeichnet, daß** es weiterhin Protcinhydrolysate und/oder deren Derivate enthält.

8. Mittel nach einem der Ansprüche 4 bis 7, **dadurch gekennzeichnet, daß** weiterhin ein Polymer enthalten ist.

9. Mittel nach einem der Ansprüche 4 bis 8, **dadurch gekennzeichnet, daß** weiterhin ein Kohlenhydrat enthalten ist.

10. Mittel nach Anspruch 9, **dadurch gekennzeichnet, daß** das Kohlenhydrat eine Onsäure oder deren Derivat oder deren Salz ist.

11. Mittel nach Anspruch 10, **dadurch gekennzeichnet, daß** das Kohlenhydrat Gluconsäure oder eines ihrer Salze ist.

12. Mittel nach einem der Ansprüche 4 bis 11, **dadurch gekennzeichnet, daß** eine Aminosäure und/oder deren Salz und/oder deren Derivat enthalten ist.

13. Mittel nach Anspruch 12, **dadurch gekennzeichnet, daß** die Aminosäure ausgewählt ist aus Arginin, Asparagin, Asparaginsäure, Histidin, Ornithin und Lysin.

## Claims

1. A method for permanently shaping keratin fibres, in which the fibres are treated before and/or after mechanical shaping with an aqueous preparation of a keratin-reducing substance, optionally rinsed after a period of exposure with a first rinse, then fixed with an aqueous preparation of an oxidising agent and rinsed again after a period of exposure and/or optionally post-treated, **characterised in that** at least one of the two aqueous preparations or the first rinse is in the form of a two-or multi-phase system, which contains at least one hydrophobic component and which may be converted by mechanical movement into a homogeneous system for application onto the fibres, wherein the hydrophobic component contains
a. at least one silicone oil with a kinematic viscosity of up to 50,000 mm²s⁻¹ (50,000 cSt) at 25°C and
b. a fatty acid and fatty alcohol ester, selected from modified cocoglycerides and/or a dialkyl carbonate,
wherein the ratios by weight of a. to b. amount to 100:1 1 to 1:1.

2. A method for permanently shaping keratin fibres according to claim 1, **characterised in that** the hydrophobic component furthermore contains at least one alcohol having only limited miscibility with water.

3. A method for permanently shaping keratin fibres according to claim 1, **characterised in that** the hydrophobic component furthermore contains at least one carbohydrate.

4. An agent for carrying out the reducing stage of a method for permanently shaping keratin fibres containing a keratin-reducing substance together with conventional constituents, **characterised in that** it is in the form of a two- or multi-phase system which contains at least one hydrophobic component which is composed of
a. at least one silicone oil with a kinematic viscosity of up to 50,000 mm²s⁻¹ (50,000 cSt) at 25°C and
b. a fatty acid and fatty alcohol ester, selected from modified cocoglycerides and/or a dialkyl carbonate, wherein the weight ratios of a. to b. amount to 100:1 1 to 1:1,
and which may be converted by mechanical movement into a homogeneous system.

5. An agent for carrying out the oxidising stage of a method for permanently shaping keratin fibres containing an oxidising agent together with conventional constituents, **characterised in that** it is in the form of a two-or multi-phase system which contains at least one hydrophobic component which is composed of
a. at least one silicone oil with a kinematic viscosity of up to 50,000 mm²s⁻¹ (50,000 cSt) at 25°C and
b. a fatty acid and fatty alcohol ester, selected from modified cocoglycerides and/or a dialkyl carbonate, wherein the weight ratios of a. to b. amount to 100:1 1 to 1:1,
and which may be converted by mechanical movement into a homogeneous system.

6. An agent for rinsing after carrying out the reducing stage of a method for permanently shaping keratin fibres containing conventional constituents, **characterised in that** it is in the form of a two- or multi-phase system which contains at least one hydrophobic component which is composed of
a. at least one silicone oil with a kinematic viscosity of up to 50,000 mm²s⁻¹ (50,000 cSt) at 25°C and
b. a fatty acid and fatty alcohol ester, selected from modified cocoglycerides and/or a dialkyl carbonate, wherein the weight ratios of a. to b. amount to 100:1 to 1:1,
and which may be converted by mechanical movement into a homogeneous system.

7. An agent according to any one of claims 4 to 6, **characterised in that** it furthermore contains protein hydrolysates and/or the derivatives thereof.

8. An agent according to any one of claims 4 to 7, **characterised in that** a polymer is furthermore contained therein.

9. An agent according to any one of claims 4 to 8, **characterised in that** a carbohydrate is furthermore contained therein.

10. An agent according to claim 9, **characterised in that** the carbohydrate is an onic acid or the derivative thereof or the salt thereof.

11. An agent according to claim 10, **characterised in that** the carbohydrate is gluconic acid or one of the salts thereof.

12. An agent according to any one of claims 4 to 11, **characterised in that** an amino acid and/or the salt thereof and/or the derivative thereof is contained therein.

13. An agent according to claim 12, **characterised in that** the amino acid is selected from arginine, asparagine, aspartic acid, histidine, ornithine and lysine.

## Revendications

1. Procédé pour la mise en forme durable de fibres kératiniques, dans lequel on traite les fibres avant et/ou après une mise en forme mécanique par une préparation aqueuse d'une substance réductrice de la kératine, on les rince éventuellement après une durée d'action avec un premier rinçage, puis on les fixe avec une préparation aqueuse d'un agent d'oxydation et on les rince également après une durée d'action et/ou on les traite éventuellement a posteriori, **caractérisé en ce qu'**au moins l'une des deux préparations aqueuses ou le premier rinçage existent sous forme d'un système biphasé ou multiphasé, qui contient au moins un composant hydrophobe et qui est transformé en un système homogène pour application sur les fibres à l'aide d'un mouvement mécanique, le composant hydrophobe contenant
a. au moins une huile de silicone ayant une viscosité cinématique allant jusqu'à 50 000 mm²s⁻¹ (50 000 cSt) à 25°C et
b. un ester d'acide gras et d'alcool gras, choisi parmi les cocoglycérides modifiés, et/ou un carbonate de dialkyle,
les rapports pondéraux de a. à b. étant de 100:1 à 1:1.

2. Procédé pour la mise en forme durable de fibres kératiniques selon la revendication 1, **caractérisé en ce que** le composant hydrophobe contient en outre au moins un alcool ayant seulement une miscibilité limitée avec l'eau.

3. Procédé pour la mise en forme durable de fibres kératiniques selon la revendication 1, **caractérisé en ce que** le composant hydrophobe contient en outre au moins un glucide.

4. Agent pour réaliser l'étape réductrice d'un procédé pour la mise en forme durable de fibres kératiniques contenant une substance réductrice de la kératine ainsi que des composants courants, **caractérisé en ce qu'**il existe sous forme d'un système biphasé ou multiphasé, qui contient au moins un composant hydrophobe, lequel se compose
a. d'au moins une huile de silicone ayant une viscosité cinématique allant jusqu'à 50 000 mm²s⁻¹ (50 000 cSt) à 25°C et
b. d'un ester d'acide gras et d'alcool gras choisi parmi les cocoglycérides modifiés, et/ou d'un carbonate de dialkyle, les rapports pondéraux de a. à b. étant 100:1 à 1:1,
et qui peut être transformé en un système homogène au moyen d'un mouvement mécanique.

5. Agent pour réaliser l'étape d'oxydation d'un procédé pour la mise en forme durable de fibres kératiniques contenant un agent d'oxydation ainsi que des composants courants, **caractérisé en ce qu'**il existe sous forme d'un système biphasé ou multiphasé, qui contient au moins un composant hydrophobe, lequel se compose
a. d'au moins une huile de silicone ayant une viscosité cinématique allant jusqu'à 50 000 mm²s⁻¹ (50 000 cSt) à 25°C et
b. d'un ester d'acide gras et d'alcool gras choisi parmi les cocoglycérides modifiés, et/ou d'un carbonate de dialkyle, les rapports pondéraux de a. à b. étant 100:1 à 1:1,
et qui peut être transformé en un système homogène au moyen d'un mouvement mécanique.

6. Agent pour le rinçage après réalisation de l'étape réductrice d'un procédé pour la mise en forme durable de fibres kératiniques, contenant des composants courants, **caractérisé en ce qu'**il existe sous forme d'un système biphasé ou multiphasé, qui contient au moins un composant hydrophobe, lequel se compose
a. d'au moins une huile de silicone ayant une viscosité cinématique allant jusqu'à 50 000 mm²s⁻¹ (50 000 cSt) à 25°C et
b. d'un ester d'acide gras et d'alcool gras, choisi parmi les cocoglycérides modifiés, et/ou d'un carbonate de dialkyle, les rapports pondéraux de a. à b. étant 100:1 à 1:1,
et qui peut être transformé en un système homogène au moyen d'un mouvement mécanique.

7. Agent selon l'une quelconque des revendications 4 à 6, **caractérisé en ce qu'**il contient en outre des hydrolysats protéiniques et/ou leurs dérivés.

8. Agent selon l'une quelconque des revendications 4 à 7, **caractérisé en ce qu'**un polymère est contenu en sus.

9. Agent selon l'une quelconque des revendications 4 à 8, **caractérisé en ce qu'**un glucide est contenu en sus.

10. Agent selon la revendication 9, **caractérisé en ce que** le glucide est un acide onique ou son dérivé, ou son sel.

11. Agent selon la revendication 10, **caractérisé en ce que** le glucide est un acide gluconique ou l'un de ses sels.

12. Agent selon l'une quelconque des revendications 4 à 11, **caractérisé en ce qu'**un acide aminé et/ou son sel, et/ou son dérivé, sont contenus.

13. Agent selon la revendication 12, **caractérisé en ce que** l'acide aminé est choisi parmi l'arginine, l'asparagine, l'acide aspartique, l'histidine, l'ornithine et la lysine.
